# EUROPEAN PATENT SPECIFICATION

(11) **EP 0 782 843 B1**
(45) Date of publication and mention of the grant of the patent: **31.07.2002**
(21) Application number: 96203424.5
(22) Date of filing: 03.12.1996
(51) Int. Cl.: A61H 1/00, A61F 5/01

(54) **Orthopedic rehabilitation apparatus using virtual reality units**
Orthopädische Vorrichtung zur Rehabilitation mittels virtueller Realitätseinheiten
Appareil orthopédique de réhabilitation utilisant des dispositifs de réalité virtuelle

(30) Priority: 02.01.1996 IT MI960001
(43) Date of publication of application: 09.07.1997
(73) Proprietor: Ferrati, Benito, 20026 Novate Milanese (MI) (IT)
(72) Inventor: Ferrati, Benito, 20026 Novate Milanese (MI) (IT)
(74) Representative: Arena, Giovanni

(56) References cited:
- EP-A- 0 667 603
- WO-A-89/01353
- DE-U- 29 511 509
- US-A- 4 557 257
- DATABASE WPI Section PQ, Week 9702 Derwent Publications Ltd., London, GB; Class P33, AN 97-015170 XP002053031 & JP 08 280 762 A (HIROKAWA K) , 29 October 1996

## Description

The present invention relates to an orthopedic apparatus for walking and rehabilitating disabled persons.

### State of the Prior Art

Orthopedic apparatus have been conceived for rehabilitating persons that are disabled in the motor abilities of their lower limbs (paraplegics, etc.) which are designed to allow walking for the purpose not only of improving the muscle, nerve and bone tonus, but also, and mainly, to stimulate and aid in the recovery of their motor ability.

Known orthopedic apparatus that are designed to stimulate and exercise the movement of the lower limbs and to allow walking, that have been designed by the present inventor include those disclosed in Italian patents 1,153,225, filed October 12, 1982 and 1,178,548, filed October 3, 1984.

Of particular interest is the orthopedic apparatus described in Italian patent 1,178,548. This apparatus has the aim of allowing persons who have serious injuries to their motor abilities of their lower limbs to walk. The apparatus consists of a semi-rigid apparatus or exoskeleton that supports the bust and the lower limbs. It is jointed opposite the hip and knee articulations, and is equipped with small actuators (microcylinders) that are operated by compressed air, hydraulically or electrically, and are designed to move the lower jointed parts of the apparatus in accordance with the human gait and on command of a programmed electronic unit that is in turn operated by the patient by means of a remote control.

This permits the patient to transmit to the electronic commands for starting and stopping the lower joints parts of the apparatus or to adjust the step speed.

### Summary of the Invention

The object according to the present invention is to further improve the apparatus of the prior art, and more particularly, to further strengthen the stimulation of the recovery of motor abilities and to facilitate the use of an exoskeleton by a patient.

Said object is achieved by an orthopedic apparatus comprising:
- an exoskeleton adapted to support a user's body comprising a plurality of jointed members jointed together at articulation positions corresponding to knee and hip joints of the user and actuators connected to said jointed members for moving said jointed members relative to each other in a movement corresponding to the human gait, whereby the movements of the human gait are impressed on the user;
- a programmed control unit connected to said actuators for controlling the operation of said actuators to move in accordance with the human gait;
- a remote control unit for controlling said programmed control unit with start, stop and speed commands for stopping, starting and controlling the speed of the human gait that is impressed on the user by the exoskeleton, said remote control unit comprising push buttons for the operation thereof;
- an electronic virtual reality unit connected to a virtual reality helmet for wear by the user of the exoskeleton for transmitting to the user virtual reality pictures and stimulation interactive with the human gait that is impressed on the user by the exoskeleton;
- and a framework for steadying and supporting the user when the exoskeleton impresses the human gait on the user, said framework having grips for the user.
Other particular solutions are set forth below in the claims.

### General Description of the Drawings

Further objects, features and advantages of the present invention will be apparent from the following detailed description taken in conjunction with the accompanying drawings, in which:
- Fig.1: illustrates a person using a walking apparatus of a known type;
- Fig.2: illustrates a first example of a walking and rehabilitating apparatus according to the present invention; and
- Fig.3: illustrates a second embodiment in accordance with the present invention.

### Detailed Description of the Preferred Embodiments

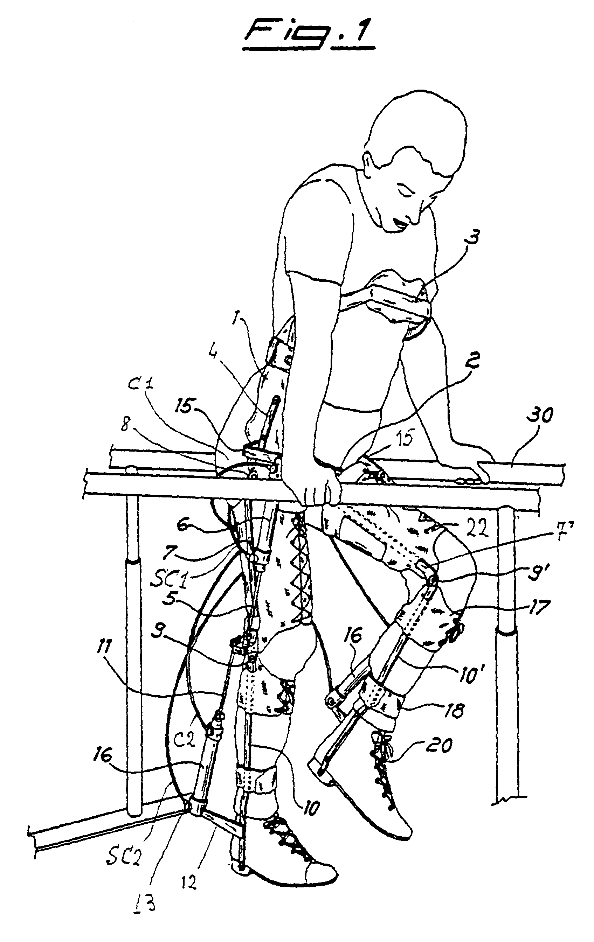

Fig. 1 illustrates a disabled person who is leaning with his hands on two fixed bars. The person is equipped with a walking apparatus of the type which was described above and is disclosed in Italian patent No. 1,178,548. This apparatus comprises a metal corset 1 that is attached to the chest and the pelvis by two attachment devices 2 and 3. On two sides of the corset are fixed metal rods 4 that each have an integral extension 15. At the lower end of the rod 4, at a hinge point 8, there is attached a rod 7. The rod 7 extends along the external side of the thigh. A corresponding rod 7' is arranged parallel on the internal side of the thigh, and together with the rod 7 forms a framework of the thigh. A cylinder 6 operated by compressed air or hydraulically is hingedly connected to an end of the extension 15 at an upper end thereof. The cylinder 6 has a rod 5 having a lower end that is connected through a joint to a lower end of the rod 7.

The rod 7 has a lower end that terminates at a hinge 9 that connects the rod 7 with a lower rod 10. The lower rod 10 is part of the leg framework. On the lower part of the rod 10 there is fixed an integral extension 12. The extension 12 is shown enlarged in the drawing in order to provide greater clarity.

A cylinder 16 is connected and hinged to an end of the extension 12, and has a rod 11 that is connected through a joint to a small bracket that is fixed to the lower end of the rod 7. There is a corresponding rod 10' similarly hinged together with the rod 7' for the inner side of the leg.

A plurality of laced bands 17, 18 and 22 allow the rods 7 and 7' and 10 and 10' to be tightened to the thighs and legs of the patient.

Extension and retraction of the rod 5 of the cylinder 6 causes relative movement between the thigh and the pelvis. Extension and retraction of the rod 11 causes relative movement between the thigh and the lower leg. The cylinders 6 and 16, which are microcylinders, are operated by tubes C1, C2, SC1 and SC2, which provide for the inlet and outlet of a working fluid such as compressed air or hydraulic fluid. The patient wears on his back a pack (not illustrated) in which are housed compressed air bottles, for example, for operating the cylinders.

Solenoid valves are provided for operating the microcylinders. They are controlled by an electronically or hydraulically operated control unit that is fixed to the patient's belt, and appropriately programmed in order to move the lower jointed parts of the device in accordance with the human gait. The control unit is in turn controlled by means of a manually operated remote control which is located on the patient's corset or belt, and that permits the patient to transmit to the control unit commands for starting or stopping the movement of the lower parts or for adjusting the step speed.

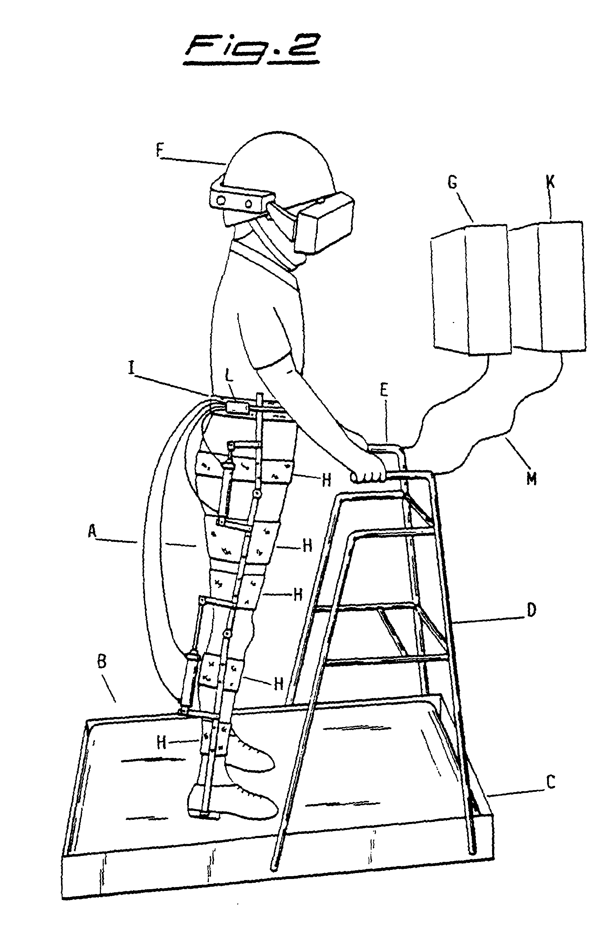

Fig. 2 illustrates a disabled person employing a rehabilitation apparatus in accordance with a first embodiment of the present invention. This apparatus has a sling or exoskeleton A that is laced to the person or patient's body. The sling is similar to that illustrated in Fig. 1, but is improved over the sling of Fig. 1 as will be explained below.

A conveyor belt B provides a support for the patient's feet, and has a rectangular frame C that surrounds the belt and supports rollers of the conveyor belt B. A tubular framework D is fixed to the rectangular frame C, and is designed to act mainly as a support for the patient through two upper grips E.

A helmet F is worn by the patient, and is provided with earphones and viewers that are connected to an electronic virtual reality unit G.

The sling or exoskeleton that is shown in Fig. 2 is improved with respect to that of Fig. 1 by making the frame members that were previously metallic parts out of an ultralight material such as a carbon fiber and titanium. This reduces the weight on the patient's body in movement and permits an independent wearing of the exoskeleton or sling by the patient, in contrast to the sling or exoskeleton of Fig. 1. Such independent wear of the sling or exoskeleton is further facilitated by the use of bands H employing hooks and loop type fasteners such as Velcro. It is further improved by, for patients who are capable of holding their chest erect, a corset that is essentially limited to a belt I.

The control unit (not shown), in addition to controlling the operation of the solenoid valves, which are enclosed in a distribution box L provided on a belt I, controls movement of the conveyor belt. It is programmed to regulate the running speed of the conveyor belt in dependence upon the speed of the walking movement of the exoskeleton.

Instead of being placed on the patient's belt, the remote control operating push buttons are, with this embodiment, located on the hand grips E of the tubular frame D. This allows the patient to timely operate the remote control during any phase of walking.

The precise positioning of the operating push buttons is not illustrated in the figures.

Furthermore, a source of pressurized fluid, such as compressed air bottles, are no longer located on the patient's back. This is because they can be located in a special delivery unit K having a delivery duct M. The duct M is appropriately connected to the framework, for example, and can have a passage extending through the framework, or at least an anchoring point on the framework D.

As an alternative, the source of pressurized fluid, or delivery means, could be located on the framework D itself, comprising either a simple compressed gas (for example air) bottle, or a compressor, or a pump that provides hydraulic operation of the microcylinders.

The special delivery unit K and its delivery duct M, whatever its configuration, of course has a fluid passage or connection to the distribution box L for operation of the microcylinders.

The cable that connects the electronic virtual reality unit G to the patient's helmet can also pass through the framework E, or at least have an appropriate anchor point on the framework D. The virtual reality unit G is programmed to transmit to the patient virtual reality pictures and stimulation that is interactive with the walking movement that is impressed by the exoskeleton on the patient's body. This involves the patient emotionally, which is important, because therapeutic efficacy is proportional to the emotional involvement of the patient. Examples of the types of virtual reality pictures and stimulation are pictures and stimulation that are related to the path which, according to the pictures projected in the helmet, the patient believes he is following, and can reflect situations of emergency and danger that occur along the path, or pictures and stimulation that are related to paths that are particularly dear or at least significant in the memory of the patient. Sounds and voices that can be either danger signals, or sounds, music and voices that are particularly dear to the patient, can also be provided.

In order to explain the utility of the equipment in accordance with the present invention, and in particular the use of virtual reality with the present invention, the following observations are pertinent.

It is known that lesions or amputations of the spinal medulla due to injuries to the spinal column, inflammation of the medulla or the vertebra, tumors or serious vascular disorders can cause paralysis of the lower limbs due to the fact that the lesions constitute a break in the transmission of nervous motor impulses from the brain. It is also known that the motor program of the limbs is not located only in the brain, but also in the spinal medulla, as proven by the fact that animals run on their own legs for a few moments even after amputation of the head. The above observations serve as a key for the interpretation of rehabilitation methods that are based on a program of exercise for the patient in walking in a manner as natural as possible with the aid of special support and motor mechanisms for the legs. These methods allow not only the reactivation or rememorization of the motor program of the legs in the spinal medulla, but also progressively reactivate the passage of the nervous impulses from the brain in the injured zone of the medulla. The usefulness of virtual reality in this situation is based on the properties of adrenaline and noradrenaline and on the fact that the secretion of adrenaline is stimulated upon the occurrence of emergency situations such as strenuous efforts, emotions, danger signals, arterial hypotension, asphyxia, hypothermia, etc. The growth of the concentration of adrenaline in the blood can be up to levels that are 300 times normal.

The adrenaline and noradrenaline hormones have the function of preparing the system both physically and emotionally to face emergency situations.

Among their various effects is a stimulation of the nervous system.

In particular, adrenaline and noradrenaline act as neurotransmitters. That is, they are chemical intermediaries that allow the transmission of nervous impulses from one neuron to another.

It is thus understandable how useful virtual reality could be during a patient's walking exercises as a means of stimulating the transmission of nervous impulses in the injured zone of the medulla through virtual reality pictures, sensations and emotions that are designed to stimulate the increase of the adrenaline levels in the blood.

It could be advantageous to use the apparatus according to the present invention without the use of the virtual reality helmet, either before starting therapy with the helmet, in order to allow the patient to gain familiarity and practice in the use of the conveyor belt, or after the therapy cycle with the helmet, when the voluntary mobility of the lower limbs has begun to appear.

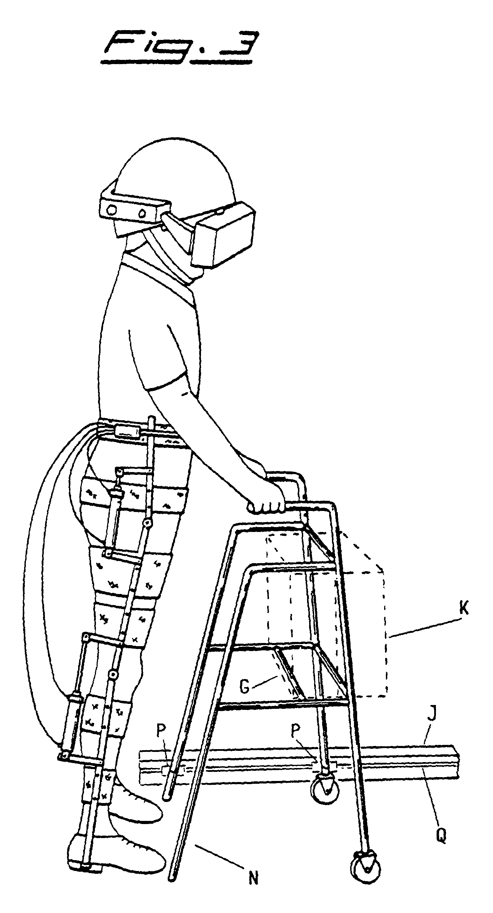

Fig. 3 is an illustration of a disabled person using a rehabilitation apparatus according to a second embodiment of the present invention.

The main difference between the example of Fig. 3 and that of Fig. 2 is the lack of the conveyor belt, and the conversion of the tubular frame into a four legged hand walker N with the front legs thereof being fitted with wheels. With this type of arrangement, the patient can move around in the room.

In order to facilitate the walking of the patient, it may be appropriate to install in the room where the walking exercises take place a rail J that is equipped with slides P running along a groove Q in the rail.

The slides P are firmly fixed through rings, for example, to the two legs of the hand walker that are on one side thereof, so that the walking of the patient is guided. The constraint of the use of the walker along the rail can be removed as soon as the rehabilitation has progressed to the point where use of the virtual reality helmet is no longer required.

The electronic virtual reality unit G and the pressurized fluid delivery unit K can still be located in fixed positions, as shown in Fig. 2, or they could be installed in the frame N, as shown in broken lines in Fig. 3.

It will be clear to those of skill in the art that numerous modifications, adaptations, variations and substitutions of various elements through other elements can be made to the above-described embodiments. For example, the two slides P that are illustrated in Fig. 3 could be replaced by a single slide that is fixed to one side of the frame N.

## Claims

1. An orthopedic apparatus for walking and rehabilitating disabled persons, comprising:
- an exoskeleton (A) adapted to support a user's body comprising a plurality of jointed members jointed together at articulation positions corresponding to knee and hip joints of the user and actuators connected to said jointed members for moving said jointed members relative to each other in a movement corresponding to the human gait, whereby the movements of the human gait are impressed on the user;
- a programmed control unit connected to said actuators for controlling the operation of said actuators to move said jointed members in accordance with the human gait;
- a remote control unit for controlling said programmed control unit with start, stop and speed commands for stopping, starting and controlling the speed of the human gait that is impressed on the user by the exoskeleton, said remote control unit comprising push buttons for the operation thereof;
- and a framework (D) for steadying and supporting the user when the exoskeleton impresses the human gait on the user, said framework having grips (E) for the user.
**characterised in that** the apparatus further comprises
- an electronic virtual reality unit (G) connected to a virtual reality helmet (F) for wear by the user of the exoskeleton for transmitting to the user virtual reality pictures and stimulation interactive with the human gait that is impressed on the user by the exoskeleton;

2. The orthopedic apparatus of claim 1, further comprising a conveyor belt for supporting the user during the impression of the human gait on the user by the exoskeleton, said conveyor belt having a support frame and said framework being fixed to said support frame.

3. The orthopedic apparatus of claim 2, wherein said programmed control unit is connected with said conveyor belt for control of said conveyor belt thereby such that the speed of said conveyor belt corresponds to the speed of the human gait impressed by said exoskeleton on the user.

4. The orthopedic apparatus of claim 1, further comprising a guide rail fixed to a floor and at least one slide that is slidable along said guide rail, wherein said framework comprises four legs, two of said four legs are front legs that have wheels thereon, and two of said four legs are side legs that are fixed to said at least one slide.

5. The orthopedic apparatus of claim 1, further comprising a guide rail fixed to a wall and at least one slide that is slidable along said guide rail, wherein said framework comprises four legs, two of said four legs are front legs that have wheels thereon, and two of said four legs are side legs that are fixed to said at least one slide.

6. The orthopedic apparatus according to one of claim 2, 4, 5 wherein said actuators comprise fluid cylinders connected to a source of pressurized fluid, and wherein said push buttons are mounted on said grips.

7. The orthopedic apparatus according to claim 6, wherein said source of pressurized fluid and said virtual reality unit are mounted on said framework.

## Patentansprüche

1. Orthopädisches Lauf- und Rehabilitationsgerät für behinderte Personen mit:
- einer Außenschiene (A), die sich zur Abstützung des Körpers eines Benutzers eignet, mit einer Mehrzahl von gelenkig miteinander verbundenen Elementen, die an Gelenkpositionen entsprechend Knie- und Hüftgelenken des Benutzers zusammengelenkt sind und mit Aktuatoren für die Bewegung der gelenkverbundenen Glieder relativ zueinander in einer dem menschlichen Gehverhalten entsprechenden Bewegung verbunden sind, wobei dem Benutzer die menschlichen Gehbewegungen aufgeprägt werden,
- einer programmierten Steuereinheit, die mit den Aktuatoren zur Steuerung von deren Bewegung für die Bewegung der zusammengelenkten Glieder entsprechend der menschlichen Gehbewegung verbunden ist,
- einer Fernsteuereinheit zur Steuerung der programmierten Steuereinheit mit Start-, Stopp- und Geschwindigkeitsbefehlen zum Stoppen, Starten und Steuern der Bewegung der menschlichen Gehbewegung, welche dem Benutzer durch die Außenschiene aufgeprägt wird, wobei die Fernsteuereinheit Drucktasten für ihre Betätigung aufweist,
- und mit einem Gestell (D) zur Stützung und zum Halten des Benutzers, wenn die Außenschiene ihm die menschliche Gehbewegung aufprägt, wobei das Gestell Griffe (E) für den Benutzer aufweist,
**dadurch gekennzeichnet, dass** das Gerät weiterhin aufweist:
- eine elektronische Virtuell-Realitäts-Einheit (G), die mit einem Virtuell-Realitäts-Helm (F) verbunden ist, den der Benutzer der Schiene trägt zur Übertragung von Bildern virtueller Realität und von Stimulationen in Interaktion mit der menschlichen Gehbewegung, die dem Benutzer durch die Außenschiene aufgeprägt wird.

2. Orthopädisches Gerät nach Anspruch 1, weiterhin mit einem Laufband, von welchem der Benutzer beim Aufprägen der menschlichen Gehbewegung durch die Außenschiene getragen wird und welches einen mit dem Gestell fest verbundenen Tragrahmen aufweist.

3. Orthopädisches Gerät nach Anspruch 2, bei welchem die programmierte Steuereinheit mit dem Laufband zu dessen Steuerung derart verbunden ist, dass die Laufbandgeschwindigkeit der Geschwindigkeit der dem Benutzer von der Außenschiene aufgeprägten menschlichen Gehbewegung entspricht.

4. Orthopädisches Gerät nach Anspruch 1, weiterhin mit einer am Boden befestigten Führungsschiene und mindestens einem Gleiter, der entlang der Führungsschiene geführt wird, wobei das Gestell vier Beine aufweist, von denen zwei Vorderbeine mit Rädern sind und zwei Seitenbeine sind, die mit dem mindestens einem Gleiter fest verbunden sind.

5. Orthopädisches Gerät nach Anspruch 1, weiterhin enthaltend eine an einer Wand befestigte Führungsschiene und mindestens einen Gleiter, der entlang dieser Führungsschiene geführt wird, wobei das Gestell vier Beine aufweist, von denen zwei Vorderbeine mit Rädern und zwei Seitenbeine sind, die mit dem mindestens einen Gleiter fest verbunden sind.

6. Orthopädisches Gerät nach einem der Ansprüche 2, 4 und 5, bei welchem die Aktuatoren Fluidzylinder sind, die mit einer Druckfluidquelle verbunden sind, und bei welchem an den Griffen Drucktasten montiert sind.

7. Orthopädisches Gerät nach Anspruch 6, bei welchem die Druckfluidquelle und die Virtuell-Realitäts-Einheit an dem Gestell montiert sind.

## Revendications

1. Appareil orthopédique pour faire marcher et réhabiliter des personnes handicapées, comprenant :
un exosquelette (A) propre à supporter le corps d'un utilisateur, comprenant une pluralité de membres articulés les uns par rapport aux autres en des positions d'articulation correspondant aux articulations du genou et de la hanche de l'utilisateur et des actionneurs reliés aux membres articulés pour déplacer les membres articulés les uns par rapport aux autres selon un mouvement correspondant à la démarche humaine, d'où il résulte que les mouvements de la démarche humaine sont imprimés à l'utilisateur ;
un module de commande programmé relié aux actionneurs pour commander le fonctionnement des actionneurs pour déplacer les membres articulés en accord avec la démarche humaine ;
un module de commande à distance pour commander le module de commande programmé avec des commandes de démarrage, d'arrêt et de vitesse pour arrêter, démarrer et commander la vitesse de la démarche humaine qui est imprimée à l'utilisateur par l'exosquelette, le module de commande à distance comprenant des boutons poussoirs pour son actionnement ; et
un bâti (D) pour stabiliser et supporter l'utilisateur lorsque l'exosquelette imprime la démarche humaine à l'utilisateur, le bâti comprenant des poignées (E) pour l'utilisateur ;
**caractérisé en ce que** l'appareil comprend en outre :
un module électronique de réalité virtuelle (G) relié à un casque de réalité virtuelle (F) propre à être porté par l'utilisateur de l'exosquelette pour transmettre à l'utilisateur des stimuli et des images de réalité virtuelle de manière interactive avec la démarche humaine qui est imprimée à l'utilisateur par l'exosquelette.

2. Appareil orthopédique selon la revendication 1, comprenant en outre un tapis roulant destiné à supporter l'utilisateur lors de l'impression de la démarche humaine à l'utilisateur par l'exosquelette, le tapis roulant comprenant un châssis de support et le bâti étant fixé au châssis de support.

3. Appareil orthopédique selon la revendication 2, dans lequel le module de commande programmé est relié au tapis roulant pour commander le tapis roulant de telle manière que la vitesse du tapis roulant correspond à la vitesse de la démarche humaine imprimée par l'exosquelette à l'utilisateur.

4. Appareil orthopédique selon la revendication 1, comprenant en outre un rail de guidage fixé à un sol et au moins un élément coulissant qui peut coulisser dans le rail de guidage, dans lequel le bâti comprend quatre pieds, deux des quatre pieds étant des pieds avant comportant des roues, et deux des quatre pieds étant des pieds latéraux fixés audit au moins un élément coulissant.

5. Appareil orthopédique selon la revendication 1, comprenant en outre un rail de guidage fixé à un mur et au moins un élément coulissant qui peut coulisser dans le rail de guidage, dans lequel le bâti comprend quatre pieds, deux des quatre pieds étant des pieds avant comportant des roues, et deux des quatre pieds étant des pieds latéraux fixés audit au moins un élément coulissant.

6. Appareil orthopédique selon l'une des revendications 2, 4, 5, dans lequel les actionneurs comprennent des vérins reliés à une source de fluide sous pression, et dans lequel les boutons poussoirs sont disposés sur les poignées.

7. Appareil orthopédique selon la revendication 6, dans lequel la source de fluide sous pression et le module de réalité virtuelle sont disposés sur le bâti.
